# EUROPEAN PATENT SPECIFICATION

(11) **EP 4 019 008 B1**
(45) Date of publication and mention of the grant of the patent: **09.10.2024**
(21) Application number: 20216961.1
(22) Date of filing: 23.12.2020
(51) Int. Cl.: A61K 9/20

(54) **NATURAL GLIDANT COMPOSITIONS**
NATÜRLICHE GLEITMITTELZUSAMMENSETZUNGEN
COMPOSITIONS GLISSANTES NATURELLES

(43) Date of publication of application: 29.06.2022
(73) Proprietor: Bonutra AG, 6312 Steinhausen (CH); Herbitat Lifesciences, LLP, Mumbai 400055 (IN)
(72) Inventor: Patel, Ritesh, Somerset, NJ New Jersey 08873 (US); Ghatak, Somsuvra, Somerset, NJ New Jersey 08873 (US); Specht, Felix, Shelbyville, KY Kentucky 40065 (US); Yunis, Mahmud, 65203 Wiesbaden (DE)
(74) Representative: Uexküll & Stolberg

(56) References cited:
- US-A1- 2013 296 445
- US-A1- 2016 008 310
- DARE KUNLE ET AL: "Effects of Pigeon Pea and Plantain Starches on the Compressional, Mechanical, and Disintegration Properties of Paracetamol Tablets", JOURNAL DRUG DEVELOPMENT AND INDUSTRIAL PHARMACY, vol. 32, no. 3, 1 January 2006 (2006-01-01), US, pages 357 - 365, XP055810354, ISSN: 0363-9045, Retrieved from the Internet <URL:https://www.tandfonline.com/doi/pdf/10.1080/03639040500519235?needAccess=true> DOI: 10.1080/03639040500519235

## Description

### BACKGROUND OF THE INVENTION

### FIELD OF THE INVENTION

The invention relates to glidant compositions, especially to natural glidant compositions which are useful in the manufacture of oral solid dosage forms such as tablets.

### DESCRIPTION OF RELATED ART

Tablets and capsules are the most accepted oral dosage forms among pharmaceutical and/or nutritional products. Generally, tablets are manufactured by compressing a suitable powder blend in a die at high compression force. There are several accepted methods for manufacturing tablets. In the direct compression method of tablets, a powder blend containing the active and excipients is directly compressed into tablets. This method is seen as the most efficient and desirable method as is does not require any pre-processing steps. Appropriate choice of the blend components is extremely critical for direct compression tablets.

Other methods of manufacturing tablets involve granulation techniques. Granulation techniques may involve wet granulation or dry granulation or slugging methods, depending on whether binding agents are added in liquid form or in dry form. Granulation techniques may be required in case actives or other excipients exhibit poor fluidity and compressibility. In those case granules with acceptable flow and compression properties have to be formed prior to tableting.

In the pharmaceutical and food industries, a number of excipients are used to manufacture solid dosage forms. These excipients are widely involved in the improvement of flowability, compression characteristics, binding and disintegration properties for enhancing the hardness of tablets or enhancing the process capability of solid dosage forms. The usage of excipients in solid dosage formulations was to perform as inert substance to deliver the necessary dosage uniformity and volume for the precise administration of the active ingredient. However, in current solid dosage forms, excipients often provide multi-functional roles such as enhancing the flowability of the powder blend, improving of patient acceptability and ensuring process performance.

Excipients are used in the manufacture of oral solid dosage forms usually in the form of fillers, disintegrants, lubricants, glidants and/or binders. The characteristics of the powders, such as flowability, filling ability and compressibility, not only affect the mixing and transfer of the powders in tableting or capsule filling process, but also affect the quality indicators for medicinal and/or nutritional preparation such as the weight variation and the uniformity of the content.

For producing high-quality tablets by direct compression a powder blend having good properties regarding homogeneity, flowability and compressibility is required. As stated, if the powder mixture does not have these properties it has to be pre-processed by granulation. However, it is preferred to utilize direct compression for the manufacture of tablets for the reasons explained above. Under the conditions that some of the bulk powders are of poor flowability or poor compressibility, it is mandatory to choose excipients with good flowability, filling and compressibility to enhance the powder properties in addition improving equipment performance.

Glidants are excipients that reduce inter-particulate friction, resulting in improved flow of granules and powders. Glidants play and significant role in improving the production process bearing in mind the weight accuracy and uniformity required by the pharmacopeias or good manufacturing practice, it thus becomes evident that controlling the flow of powders is of extreme importance. During the production of bulk powders, tablets, and capsules, efficient glidants are required to enhance the flow of granules through the feed mechanisms such as from the hopper and, ultimately, as the case may be, into the packaging chamber, tablet die, or capsule shell fill chamber.

Known artificial glidants include, for example, colloidal silicon dioxide, talc, kaolin etc. However, for health and safety concerns, artificial glidants have become undesirable. In recent years, as consumer awareness in health and natural foods has increased, the utilization of natural additives instead of artificial additives in the food and pharma industry has progressively increased, and the variety of usage thereof also has continued to gain interests. In terms of consumer preference, as it relates to pharmaceutical dosage forms and health functional foods, wellbeing, health and ecologically friendly factors in manufacturing processes are reflected more and more important, and consequently many products comprising natural components are being established and the market share of these naturally constituted products is also growing. In recent years, there has been an incredible growth in natural product developments, which are essential to be used for a variety of purposes.

Recent trend towards the use of natural and non-toxic materials therefore also requires the substitution of the existing synthetic exipients such as gliders with natural ones, while the tableting properties should be maintained.

US 2013/296445 A1 discloses a natural lubricant, that is bean powder, for direct tablet compression and a method for preparing a synthetic additive-free natural tablet using the same. 1

The systematic scanning of new natural materials for potential use as glidants in solid dosage formulations therefore continues to be of interest for the industry. This is for the reason that different glidant agents or mixtures of glidant agents can be beneficial in succeeding in enhanced process performance for different applications. The right choice of the glidant or of the glidant mixture for the development of solid dosage forms requires broad knowledge of powder properties for enhancing the robustness of the formulation, particularly tablets and capsules.

Nevertheless, it is understood that efforts at formulating all-natural or substantially all-natural nutritional supplements in a solid form contained exclusively or almost exclusively certified organic/naturally ingredients have generated intolerable outcomes. In particular, these developments have not ended up in tablets ensuring reliable characteristics, like acceptable tablet hardness, good friability and/or acceptable dissolution profile of the active ingredient. Moreover the use of natural ingredients can result in adverse processing characteristics, such as reduced flowability, poor compressibility of the powder blend and batch to batch variation of finished product.

To the knowledge of the authors, there have not been any described or commercially existing natural glidant systems in powder forms available that are comprised entirely or almost entirely of natural, certified organic components prior to this invention. And therefore, there is a requirement for improved glidant agents comprised entirely or almost entirely of natural, certified organic ingredients.

### SUMMARY OF THE INVENTION

The present invention provides an all-natural glidant pre-mix composition which provides good balance of compressibility and flowability to a powder blend during manufacture of an oral solid dosage form. In particular, the all-natural glidant composition according to the invention provides a balance of compressibility and flowability on the ingredients of solid dosage forms to enhance process performance of capsule filling or tableting machines. This ensures the process performance of powder blends in the manufacturing of oral solid dosage forms is enhanced, safeguards critical quality attributes of finished products and a clean label may be claimed.

Ultimately, the all-natural glidant pre-mix composition according to the present invention provides powder properties that enable a powder blend to be directly compressed into tablets without the need of any pre-processing steps.

The current inventors have performed several studies to overcome the mentioned challenges and to develop a natural glidant system comprising only natural component. The glidant compositions according to the present invention include a mixture consisting of pea starch powder in an amount of about 10 wt.% to about 50 wt.%, bamboo extract powder in an amount of about 40 wt.% to about 90 wt. % and bamboo fiber in an amount of about 0 wt.% to about 30 wt.%, all weight percentages being based on the total dry weight of the glidant composition. In another embodiment, the glidant compositions according to the present invention consists of pea starch powder in an amount of about 10 wt.% to about 40 wt.%, bamboo extract powder in an amount of about 50 wt.% to about 80 wt.% and bamboo fiber in an amount of about 10 wt.% to about 20 wt.%, all weight percentages being based on the total dry weight of the glidant composition **Pea Starch:** Pea starch is derived from the dry *Pisum Sativum* fruit. It is a white powder with a neutral odor and taste, low glycemic index, and low gelatinization temperature. It is normally used as a gelling or thickening agent and for its film-forming properties in certain coatings or texturized foods. The grade of the Pea Starch can be organic certified and/or according to Food Chemical Codex (FCC).

**Bamboo Extract:** Bamboo extract is derived from *Bambusa Vulgaris* which is native to Asia. The powder is normally used as exfoliant in masks and scrubs. Bamboo extract is organic certified and is rich in minerals, especially organic silica. For the purposes of the present invention bamboo extract powder containing at least 70 wt.% silica, preferably at least 75 wt.% silica, based on the dry weight of the bamboo extract powder, is preferred. The grade of the bamboo extract can be organic certified and/or Food Chemical Codex (FCC) .

**Bamboo Fiber:** Bamboo fiber is usually used in food industries and is much valued as an ingredient because of its water-binding and texturizing properties in many processed food products varying from bakery products, dairy products, meat and fish products, beverages, sauces, and dressings. Bamboo fibers have a significant position as a stabilizer in functional foods. Its function as a low-calorific additive is used to avoid moisture loss and to preserve taste and flavor of food products. The grade of the bamboo fiber can be organic certified and/or according to Food Chemical Codex (FCC) .

In a further embodiment, the present invention provides a method for manufacturing a tablet comprising physically blending an active, one or more excipients and the glidant composition according to one of the embodiments described above, the blend being substantially free of an artificial glidant, and directly compressing the so-obtained blends into a tablet. In a still another embodiment of the present invention, the glidant composition according to one of the embodiments described above is added to the blend in an amount ranging between about 0.5% and about 10% (w/w), according to a yet further embodiment an amount ranging between about 2% and about 5% (w/w) of the dry weight of the tablet constituents.

In yet another embodiment, the one or more excipients are selected from the group consisting of fillers, disintegrants, lubricants, binders and mixtures thereof. In still another embodiment, the excipients are selected form natural-source excipients.

The invention accordingly comprises the several natural components and the relation of the different components with respect to each of the others, all is exemplified in the following detailed disclosure, and the scope of the invention is defined by the scope of the appended claims.

### DETAILED DESCRIPTION OF THE INVENTION

The invention will now be described with reference to the appending Figures, wherein:
FIG. 1 is a chart setting out the calculated Hausner Factors of glidant compositions according to the invention and of comparative glidants;
FIG. 2 is a chart setting out the compressibility index of glidant compositions according to the invention and of comparative glidants.

As stated, the glidant compositions according to the present invention consist of pea starch powder in an amount of about 10 wt.% to about 50 wt.%, preferably about 10 wt.% to about 40 wt.%, bamboo extract powder in an amount of about 40 wt.% to about 90 wt.%, preferably about 50 wt.% to about 80 wt.%, and bamboo fiber in an amount of about 0 wt.% to about 30 wt.%, preferably about 10 wt.% to about 20 wt.%, all weight percentages being based on the total dry weight of the glidant composition.

The glidant composition according to the invention is preferably used as a pre-mix composition and was found to enhance both flow and compressibility of tableting formulations. The content of the glidant composition in the tablet may preferably range from about 0.5% to about 10% (w/w), preferably between about 2% to about 5% (w/w), based on the total dry weight of the tablet constituents. In these amount, the glidant composition according to the present invention is particularly effective.

### EXAMPLES

Hereinafter, the present invention will be described in detail with reference to examples. It is to be understood, however, that these examples are for illustrative purposes only.

All weight percentages are per dry weight of the composition

### EXAMPLE 1:

A dry binder premix composition according to the invention (Formula 6) has been formulated as follows:

| **Ingredients:** | **Grades:** | **% w/w** |
|---|---|---|
| Pea Starch | organic | 30 |
| Bamboo Extract (75% Silica) | organic | 50 |
| Bamboo Fiber | organic | 20 |

The dry premix has been produced by mixing of the ingredients in a dry powder blender, such as a V-type powder blender, until a homogenous mixture is achieved.

### EXAMPLE 2:

Further dry binder premix compositions according to the invention (Formulas 2-4 and 7-8) have been formulated in the same manner as in Example 1 as follows:

| **Ingredients** | **Formula 2** | **Formula 3** | **Formula 4** | **Formula 5** | **Formula 7** | **Formula 8** |
|---|---|---|---|---|---|---|
| Pea starch | 100 | 20% | 10% | 20% | 30% | 40% |
| Bamboo Extract (75% SiO₂) | 80% | 70% | 70% | 60% | 40% | 60% |
| Bamboo Fiber | 10% | 10% | 20% | 20% | 30% | 0% |

### EXAMPLE 3:

The influence of the glidants on tablet disintegration has been examined.

The glidant compositions according to Formulas 1 to 8 were blended into a tableting preparation in amounts of 2 wt.% and 5 wt.%, respectively, based on the total dry weight of the tableting composition. As the basic powder for the test series, commercially available microcrystalline powder type 102 (MCC 102) has been used. Throughout the early phase of compression, glidants are blended within the tablet formulation to improve flowability and uniformity within the die cavity of tablet presses.

Colloidal silicon dioxide ("Syloid"), which had been regarded as a superior synthetic glidant, has been blended into tableting formulations for comparative purposes. Syloid has been usually added to the tablet ingredients in amounts from 1 wt.% to 2 wt.%, based on the total dry weight of the tableting composition. The comparative data are obtained with Syloid amounts of 2 wt.%, based on the total dry weight of the tableting composition (MCC102).

The flowability and filling ability of the powders has been assessed by their compressibility indices and Hausner factors. The Hausner factor is the ratio between the tapped density and the bulk density of the powders. When the Hausner factor is greater than 1.5, the powder is viscous with poor flowability and filling ability. When the ratio is less than 1.3, it means the powder has good flowability and filling ability.

The difference between the tapped density and the bulk density divided by the tapped density equals to the compressibility index. Compressibility index can be easily calculated and through this parameter and the flowability of blend can be compared efficiently. The smaller the degree of compression, the better the flowability of the powder.

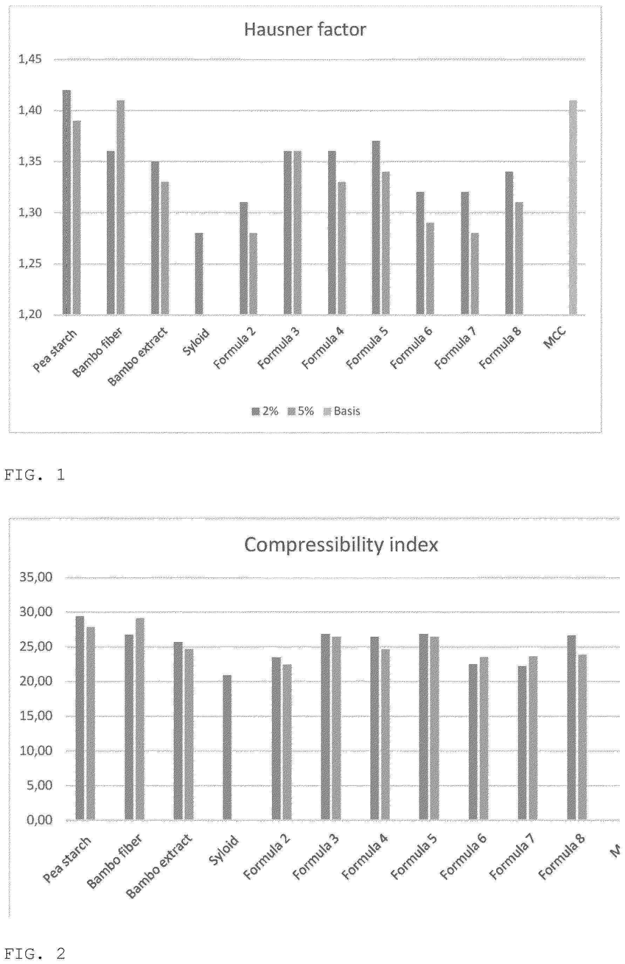

The Hausner factors of the tableting pure components - pea starch, bamboo extract and bamboo fiber, of syloid and of Formulas 1 to 8 are shown in Fig. 1. The compressibility indices of the tableting pure components - pea starch, bamboo extract and bamboo fiber, of syloid and of Formulas 1 to 8 are shown in Fig. 2.

As illustrated in the Figures, the compositions according to Formulas 2, 6 and 7 are comparable in the Hausner factors and compressibility indices to silicon dioxide ("Syloid"). The mentioned compositions according to Formulas 2, 6 and 7 decrease the Hausner factors of the basis (MCC powder) from 1.41 to under 1.3 when adding 5 wt% of the glidant pre-mixes. Regarding the compresiibility indices, a decrease from 29 for MCC to under 23 for the compositions according to Formulas 2, 6 and 7, with 5 wt.% of glidant could be observed. This decrease is comparable again with the decrease when silicon dioxide (2 wt.%) is used.

The Hausner factors and compressibility indices of Formulations 3, 4, 5 and 8 are also within an acceptable range.

## Claims

1. A glidant premix composition for the use of manufacturing of solid dosage forms consisting of pea starch powder in an amount of about 10 wt.% to about 50 wt.%, bamboo extract powder in an amount of about 40 wt.% to about 90 wt.% and bamboo fiber in an amount of about 0 wt.% to about 30 wt.%, all weight percentages being based on the total dry weight of the glidant composition.

2. The glidant composition of claim 1 consisting of pea starch powder in an amount of about 10 wt.% to about 40 wt.%, bamboo extract powder in an amount of about 50 wt.% to about 80 wt.% and bamboo fiber in an amount of about 10 wt.% to about 20 wt.%, all weight percentages being based on the total dry weight of the glidant composition.-

3. The glidant composition of any one of claims 1 to 2, wherein the grade of the pea starch is organic certified and/or according to Food Chemical Codex (FCC) .

4. The glidant composition of any one of claims 1 to 3, wherein the grade of the bamboo extract is organic certified and/or Food Chemical Codex (FCC) .

5. The glidant composition of any one of claims 1 to 4, wherein the grade of the bamboo fiber can be organic certified and/or according to Food Chemical Codex (FCC).

6. A method for manufacturing a solid oral dosage form comprising the steps of physically blending an active, one or more excipients and the glidant composition according to one or more of the previous claims, the blend being free of an artificial glidant, and directly compressing the so-obtained blends into a tablet.

7. The method of claim 6, wherein the glidant composition is added to the blend in an amount ranging between about 0.5% and about 10% (w/w).

8. The method of claim 7, wherein the glidant composition is added to the blend in an amount ranging between about 2% and about 5% (w/w).

9. An oral dosage form comprising a glidant composition according to any one of claims 1 to 5.

10. The oral dosage form of claim 9 which comprises the glidant composition in an amount ranging between about 0.5% and about 10% (w/w).

11. The oral dosage form of claim 10 which comprises the glidant composition in an amount ranging between about 2% and about 5% (w/w).

## Patentansprüche

1. Gleitmittelvormischungszusammensetzung zur Verwendung bei der Herstellung fester Dosierungsformen, bestehend aus Erbsenstärkepulver in einer Menge von etwa 10 Gew.% bis etwa 50 Gew.%, Bambusextraktpulver in einer Menge von etwa 40 Gew.% bis etwa 90 Gew.% und Bambusfasern in einer Menge von etwa 0 Gew.% bis etwa 30 Gew.%, wobei alle Gewichtsprozente auf das Gesamttrockengewicht der Gleitmittelzusammensetzung bezogen sind.

2. Gleitmittelzusammensetzung nach Anspruch 1, bestehend aus Erbsenstärkepulver in einer Menge von etwa 10 Gew.% bis etwa 40 Gew.%, Bambusextraktpulver in einer Menge von etwa 50 Gew.% bis etwa 80 Gew.% und Bambusfasern in einer Menge von etwa 10 Gew.% bis etwa 20 Gew.%, wobei alle Gewichtsprozente auf das Gesamttrockengewicht der Gleitmittelzusammensetzung bezogen sind.

3. Gleitmittelzusammensetzung nach einem der Ansprüche 1 bis 2, wobei die Qualität der Erbsenstärke biologisch zertifiziert ist und/oder dem Food Chemical Codex (FCC) entspricht.

4. Gleitmittelzusammensetzung nach einem der Ansprüche 1 bis 3, wobei die Qualität des Bambusextrakts biologisch zertifiziert ist und/oder dem Food Chemical Codex (FCC) entspricht.

5. Gleitmittelzusammensetzung nach einem der Ansprüche 1 bis 4, wobei die Qualität der Bambusfaser biologisch zertifiziert sein kann und/oder dem Food Chemical Codex (FCC) entspricht.

6. Verfahren zur Herstellung einer festen oralen Darreichungsform, umfassend in Schritten:
physikalisches Vermischen eines Wirkstoffs, eines oder mehrerer Hilfsstoffe und der Gleitmittelzusammensetzung gemäß einem oder mehreren der vorhergehenden Ansprüche, wobei die Mischung frei von einem künstlichen Gleitmittel ist, und direktes Verpressen der so erhaltenen Mischungen zu einer Tablette.

7. Verfahren nach Anspruch 6, wobei die Gleitmittelzusammensetzung der Mischung in einer Menge zwischen etwa 0,5 % und etwa 10 % (w/w) zugesetzt wird.

8. Verfahren nach Anspruch 7, wobei die Gleitmittelzusammensetzung der Mischung in einer Menge zwischen etwa 2 % und etwa 5 % (w/w) zugesetzt wird.

9. Orale Darreichungsform, umfassend eine Gleitmittelzusammensetzung gemäß einem der Ansprüche 1 bis 5.

10. Orale Darreichungsform nach Anspruch 9, die die Gleitmittelzusammensetzung in einer Menge zwischen etwa 0,5 % und etwa 10 % (w/w) enthält.

11. Orale Darreichungsform nach Anspruch 10, die die Gleitmittelzusammensetzung in einer Menge zwischen etwa 2 % und etwa 5 % (w/w) enthält.

## Revendications

1. Composition de mélange préalable d'agent d'écoulement pour l'utilisation dans la production de formes pharmaceutiques solides administrées par voie orale, constituée d'amidon de pois en poudre en une proportion d'environ 10 % en poids à environ 50 % en poids, d'extrait de bambou en poudre en une proportion d'environ 40 % en poids à environ 90 % en poids, et de fibre de bambou en une proportion d'environ 0 % en poids à environ 30 % en poids, tous les pourcentages en poids étant rapportés au poids total de matière sèche de la composition d'agent d'écoulement.

2. Composition d'agent d'écoulement selon la revendication 1, constituée d'amidon de pois en poudre en une proportion d'environ 10 % en poids à environ 40 % en poids, d'extrait de bambou en poudre en une proportion d'environ 50 % en poids à environ 80 % en poids, et de fibre de bambou en une proportion d'environ 10 % en poids à environ 20 % en poids, tous les pourcentages en poids étant rapportés au poids total de matière sèche de la composition d'agent d'écoulement.

3. Composition d'agent d'écoulement selon n'importe laquelle des revendications 1 à 2, dans laquelle l'amidon de pois est de qualité biologique certifiée et/ou conforme au codex des produits chimiques alimentaires Food Chemical Codex (FCC).

4. Composition d'agent d'écoulement selon n'importe laquelle des revendications 1 à 3, dans laquelle l'extrait de bambou est de qualité biologique certifiée et/ou conforme au codex des produits chimiques alimentaires Food Chemical Codex (FCC).

5. Composition d'agent d'écoulement selon n'importe laquelle des revendications 1 à 4, dans laquelle la fibre de bambou peut être de qualité biologique certifiée et/ou conforme au codex des produits chimiques alimentaires Food Chemical Codex (FCC).

6. Procédé de production d'une forme pharmaceutique solide administrée par voie orale, qui comporte les étapes consistant à mélanger physiquement un principe actif, un ou plusieurs excipient(s) et une composition d'agent d'écoulement conforme à une ou plusieurs des revendications précédentes, le mélange étant exempt d'agent d'écoulement d'origine synthétique, et comprimer directement le mélange ainsi obtenu sous la forme d'un comprimé.

7. Procédé selon la revendication 6, dans lequel on introduit la composition d'agent d'écoulement dans le mélange en une proportion comprise dans l'intervalle allant d'environ 0,5 % à environ 10 % (poids/poids).

8. Procédé selon la revendication 7, dans lequel on introduit la composition d'agent d'écoulement dans le mélange en une proportion comprise dans l'intervalle allant d'environ 2 % à environ 5 % (poids/poids).

9. Forme pharmaceutique administrée par voie orale, comprenant une composition d'agent d'écoulement conforme à n'importe laquelle des revendications 1 à 5.

10. Forme pharmaceutique administrée par voie orale selon la revendication 9, dans laquelle la composition d'agent d'écoulement se trouve en une proportion comprise dans l'intervalle allant d'environ 0,5 % à environ 10 % (poids/poids).

11. Forme pharmaceutique administrée par voie orale selon la revendication 10, dans laquelle la composition d'agent d'écoulement se trouve en une proportion comprise dans l'intervalle allant d'environ 2 % à environ 5 % (poids/poids).
